# EUROPEAN PATENT APPLICATION

(11) **EP 1 985 287 A2**
(43) Date of publication of application: **29.10.2008**
(21) Application number: 08251535.4
(22) Date of filing: 25.04.2008
(51) Int. Cl.: A61K 9/16, A61K 47/26, A61K 47/10, A61K 47/44

(54) **Pharmaceutical Excipient Complex**

(30) Priority: 25.04.2007 US 926282 P
(71) Applicant: Teva Pharmaceutical Industries Ltd., 49131 Petah Tiqva (IL)
(72) Inventor: Kanari, Itamar, Kefar Sava, 44235 (IL); Fox, Michael, Tel Aviv, 67291 (IL); Leibovici, Minutza, Netanya, 42312 (IL)
(74) Representative: Wong, Kit Yee

(57) **Abstract**

The present invention encompasses pharmaceutical excipient complexes comprising combining at least one carrier with an oily substance, and processes for preparing the same, stable pharmaceutical compositions comprising such complexes and active pharmaceutical ingredients and processes for preparing the same.

## Description

### CROSS-REFERENCE TO RELATED APPLICATION

This application claims the benefit of U.S. provisional application Serial No. 60/926,282, filed April 25, 2007, hereby incorporated by reference.

### FIELD OF INVENTION

The present invention encompasses pharmaceutical excipient complexes and processes for preparing the same, stable pharmaceutical compositions comprising such complexes and active pharmaceutical ingredients, and processes for preparing the same.

### BACKGROUND OF THE INVENTION

Drug formulation (immediate release, excipients used, manufacturing methods, modified release - delayed release, extended release, sustained release, etc.) can affect the bioavailability of a drug. In particular, the bioavailability of a drug can be limited by poor dissolution of the drug after being administrated non-intravenously.

Drugs with low aqueous solubility often exhibit poor dissolution rates. Examples of drugs with low aqueous solubility include, but are not limited to, albuterol, zolpidem tartrate, omeprazole, paclitaxel, quetiapine fumarate, alprostadil, candesartan, risperidone, carvedilol, celecoxib, ciprofloxacin, or alprazolam. These drugs with low aqueous solubility often need to be formulated in a manner that increases drug solubility and thus bioavailability.

Candesartan ("CNS") is an example of a drug with low aqueous solubility. Candesartan cilexetil is a white to off-white powder and is sparingly soluble in water and in methanol.

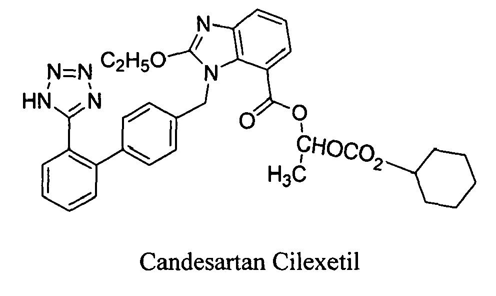

Candesartan is a potent, long-acting, selective AT₁ subtype angiotensin II receptor antagonist. Candesartan is a useful therapeutic agent for treating circulatory system diseases such as hypertensive diseases, heart diseases (e.g. hypercardia, heart failure, cardiac infarction, etc.), strokes, cerebral apoplexy, and nephritis, among others. Candesartan meets the requirement of high potency but it is poorly absorbed when administered orally. Therefore, the prodrug candesartan cilexetil was developed. During absorption in the gastrointestinal tract, candesartan cilexetil is rapidly and completely hydrolyzed to candesartan. The chemical name for candesartan is: 2-ethoxy-1-[[2'-(1H-tetrazol-5-yl)biphenyl-4-yl]methyl]-1H-benzimidazole-7-carboxylic acid. The chemical name for candesartan cilexetil is (±)-1-[[(cyclohexyloxy)carbonyl]oxy]ethyl-2-ethoxy-1-[[2'-(1H-tetrazol-5-yl)[1,1'biphenyl]-4-yl]methyl]-1H-benzimidazole-7-carboxylate.

Candesartan cilexetil is marketed in the United States under the trade name Atacand®. Atacand® tablets contain candesartan cilexetil and the following excipients: hydroxypropyl cellulose, polyethylene glycol, lactose, corn starch, carboxymethylcellulose calcium, and magnesium stearate.

European Publication No. 0459136 describes candesartan cilexetil, a process for the preparation thereof, and capsules/tablets comprising the same.

European Publication No. 0546358 relates to a method for preparing a pharmaceutical composition for oral use comprising admixing an effective amount of candesartan cilexetil with an oily substance having a low melting point (e.g. 20-90°C).

There have been many efforts directed at enhancing the rate of dissolution of the drug, for example, enhancing dissolution rates by mixing a poorly soluble drug powder with a water-soluble gelatin, which supposedly makes the surface of the drug hydrophilic (Imai, et al., J. Pharm. Pharmacol, 42:615-19 (1990)).

U.S. Patent No. 6,932,983 refers to porous drug matrices and methods of manufacture thereof, which enhance dissolution of the drug in aqueous media.

In view of the foregoing, there is a need to develop an excipient complex that can improve dissolution of the drug when incorporated into a pharmaceutical composition containing drugs with low aqueous solubility.

### SUMMARY OF THE INVENTION

One embodiment of the present invention encompasses a pharmaceutical excipient complex comprising at least one carrier intimately admixed with an oily substance.

Another embodiment of the present invention encompasses a pharmaceutical composition comprising an active pharmaceutical ingredient ("API") of low aqueous solubility and a pharmaceutical excipient complex having at least one carrier intimately admixed with an oily substance. Preferably, the active pharmaceutical ingredient is selected from the group consisting of albuterol, zolpidem tartrate, omeprazole, paclitaxel, quetiapine fumarate, alprostadil, candesartan, risperidone, carvedilol, celecoxib, ciprofloxacin, and alprazolam.

Another embodiment of the present invention encompasses a stable pharmaceutical composition comprising candesartan cilexetil and a pharmaceutical excipient complex having at least one carrier intimately admixed with an oily substance.

Yet another embodiment of the present invention encompasses a process for preparing a pharmaceutical excipient complex comprising dissolving or suspending an oily substance in a solvent to form a solution or a suspension; combining the solution or suspension with a carrier; removing the solvent; and subjecting the combination to grinding or milling.

Yet another embodiment of the present invention encompasses a process for preparing a pharmaceutical excipient complex comprising melting an oily substance; combining the melted oily substance with a carrier; cooling the combination; and grinding or milling the combination.

Yet another embodiment of the present invention encompasses a process for preparing a pharmaceutical excipient complex comprising combining a liquid oily substance with a carrier; and grinding or milling the combination.

Another embodiment of the present invention encompasses a process for preparing a pharmaceutical composition comprising combining a pharmaceutical excipient complex having at least one carrier with an oily substance; subjecting the combination to grinding or milling; and admixing the combination with an active pharmaceutical ingredient. Preferably, the process further comprises a compressing the mixture of active pharmaceutical ingredient and pharmaceutical excipient complex into a tablet.

Yet another embodiment of the present invention encompasses a method of treating a patient suffering from a disease comprising administering to a patient in need thereof a pharmaceutical composition having a therapeutically effective amount of active pharmaceutical ingredient and a pharmaceutical excipient complex having at least one carrier intimately admixed with an oily substance. Preferably, the active pharmaceutical ingredient is selected from the group consisting of albuterol, zolpidem tartrate, omeprazole, paclitaxel, quetiapine fumarate, alprostadil, candesartan, risperidone, carvedilol, celecoxib, ciprofloxacin, and alprazolam.

A further embodiment of the present invention encompasses use of an active pharmaceutical ingredient and a pharmaceutical excipient complex in the manufacture of a medicament for treating and/or preventing a patient suffering from a disease wherein the pharmaceutical excipient complex has at least one carrier intimately admixed with an oily substance. Preferably, the active pharmaceutical ingredient is selected from the group consisting of albuterol, zolpidem tartrate, omeprazole, paclitaxel, quetiapine fumarate, alprostadil, candesartan, risperidone, carvedilol, celecoxib, ciprofloxacin, and alprazolam.

### DETAILED DESCRIPTION OF THE INVENTION

Drug formulation can reduce the availability of drugs prior to their entry into the systemic circulation. In particular, drugs with low aqueous solubility exhibit poor dissolution rates. The dissolution rate of a drug particle is directly related to its surface area available to contact the aqueous media at the site of administration or at the site of absorption. The present invention provides a pharmaceutical excipient complex such that when it is formulated into a pharmaceutical composition containing drugs with low aqueous solubility, it enhances the drug particle's surface area when milling is not effective and thereby improves the drug's dissolution rate.

It will be understood that a wide variety drugs are useful in the methods and compositions described herein. In particular, the drug is preferably a low aqueous solubility drug. The term "low aqueous solubility" preferably means that the drug has a solubility of less than about 100 mg/mL, for example, less than about 50 mg/mL or less than about 20 mg/mL. More preferably the term "low aqueous solubility" refers to a drug having a solubility of less than 10 mg/mL, for example, preferably less than about 5 mg/mL, and most preferably a solubility of less than about 1 mg/mL, for example less than about 0.1 mg/mL at ambient temperature (i.e. 18-27°C).

In addition, conventional methods employed in the preparation of pharmaceutical compositions often involve one or more compression steps. Under the compression conditions, some active pharmaceutical ingredients do not exhibit good stability properties when subjected to compression, especially when wet granulated. Therefore, the present invention seeks to provide a novel pharmaceutical excipient complex that allows previously unstable active pharmaceutical ingredients to undergo compression steps during their formulation. Needless to say, the pharmaceutical excipient complex can also be used with previously stable active pharmaceutical ingredients.

In most cases, dry granulation/compression methods are preferred when preparing pharmaceutical compositions into tablets. However, dry compression methods are not feasible when an oily substance is introduced into a formulation containing an active pharmaceutical ingredient. These formulations are often mixed using wet granulation processes because the oily substance is too wet to use proper dry compression steps during formulation and in some cases the material may not be homogeneously dispersed and may form sticky formulations which require more time to fine-tune on a tablet press if at all possible.

Applicants have now developed a pharmaceutical excipient complex which is suitable for dry compression with active pharmaceutical ingredients. In most cases, the active pharmaceutical ingredients have low aqueous solubility.

As used herein, the term "oily substance" relates to a substance that is difficult or impossible to mill e.g. due to its semi solid characteristics which may make it plastic or malleable, or it may have a low glass transition temperature.

In addition to being applicable for dry compression, the pharmaceutical excipient complex stabilizes pharmaceutical compositions containing some active pharmaceutical ingredients when subjected to compression. For example, candesartan cilexetil is stable against changes in temperature, moisture and/or light when stored alone in the solid state. However, candesartan cilexetil has been observed to be unstable when it is incorporated into a pharmaceutical composition, especially a tablet, with other ingredients. Not to be limited by theory, it is believed that the loss of stability is brought about by energy introduction via, for example, pressure, abrasion and/or heat applied during the processes employed to prepare the compositions, e.g. granulation, compression or molding under elevated temperature.

It is observed that when a pharmaceutical excipient complex comprising a carrier and an oily substance compound is incorporated into a pharmaceutical composition containing active pharmaceutical ingredients, such as, candesartan cilexetil, the pharmaceutical excipient complex prevents or diminishes the active pharmaceutical ingredients from decomposing even after the pharmaceutical composition undergoes a compression step during formulation. Preferably, the oily substance is an oily substance, e.g. a poloxamer.

Not to be limited by theory, but it is believed that pharmaceutical excipient complex comprising a carrier intimately admixed with an oily substance compound absorbs the pressure, abrasion and/or heat during processing, *e.g.* during a compression step in tabletting a pharmaceutical composition having candesartan cilexetil. As a result, the pharmaceutical composition obtained after processing contains a stable candesartan cilexetil. The combination of the carrier and the oily substance also exhibits improved millability and flowability in comparison to the stabilizers employed in the prior art. Ideally, the millability and flowability of the oily substance of the present invention is similar to that of the carrier.

As used herein, the term "stable" with reference to candesartan cilexetil relates to candesartan cilexetil having about 0.01% to about 0.5% of desethyl-candesartan ("desethyl-CNS") impurity present in the total weight of the composition, after storage for four days at a temperature of 65°C and a relative humidity of 100 percent; and/or having about 0.01% to about 0.5% of 2-N-ethyl candesartan ("2-N-ethyl CNS") impurity present in the total weight of the composition, after storage for four days at a temperature of 65°C and a relative humidity of 100 percent; and/or having about 0.01% to about 0.2% of impurities other than desethyl-CNS and 2-N-ethyl CNS present in the total weight of the composition, after storage for four days at a temperature of 65°C and a relative humidity of 100 percent.

The structures of the desethyl-CNS and the 2-N-ethyl CNS impurities are as follows:

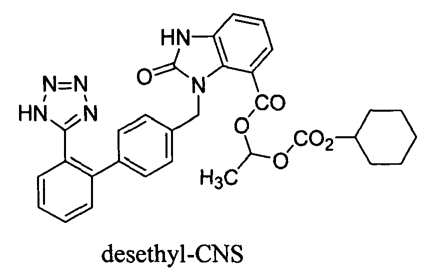

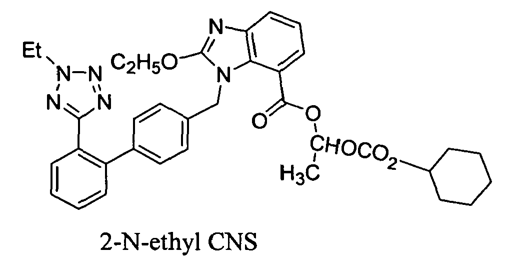

In one embodiment, the present invention encompasses a pharmaceutical excipient complex comprising at least one carrier intimately admixed with an oily substance compound. For example, the surface of the carrier can be partially or fully coated by the oily substance.

The carrier may be shaped into any form, such as particles, mini-pills, or granules. Preferably, the carrier is inert and not reactive with an active pharmaceutical excipient. As used herein, the term "inert" means not chemically active.

Suitable carriers for use in the present invention include, but are not limited to, pharmaceutically acceptable solids with good millability or flowability. Examples of such carriers include, but are not limited to lactose, white sugar, glucose, starch, calcium carbonate, kaolin, crystalline cellulose, maltose, mannitol, sorbitol, hydroxypropyl cellulose (e.g. low substituted HPC), maltodextrin, dibasic calcium phosphate or silicon dioxide. In some cases, the carrier may be a mixture of suitable carriers, i. e. a mixture of two or more carriers. Preferably, the carrier is selected the group consisting of starch, lactose monohydrate, mannitol, sorbitol, and mixtures thereof. More preferably, the carrier is starch and/or lactose monohydrate.

Typically, the oily substance is inert. Suitable oily substances include, but are not limited to, waxes, polymers, block co-polymers, stabilizers, surfactants, fats, and fatty acids. Preferably, the oily substance is poloxamer, glyceryl behenate, bees wax, glyceryl stearate, stearyl alcohol, hydrogenated castor oil, liquid triglyceride, or mixture thereof. More preferably, the oily substance is a poloxamer.

A poloxamer is a block copolymer of ethylene oxide and propylene oxide. Poloxamers are often referred to as polyethylene-propylene glycol copolymers or polyoxyethylene-polyoxypropylene copolymers. Preferably, the poloxamer has an average molecular weight of about 9800 to about 14000. Commercially available poloxamers include Lutrol^{®} F127 (Poloxamer 407, available from BASF corporation).

In the pharmaceutical excipient complex, the weight ratio between the oily substance and the carrier is in the range of about 1:100 to about 1:1. Preferably, it is in the range of about 1:50 to about 1:3. The suitable amount of each active pharmaceutical ingredient can be determined based upon the disease being treated and the therapeutic effect desired.

In another embodiment, the present invention encompasses a process for preparing a pharmaceutical excipient complex comprising dissolving or suspending the oily substance in a solvent to form a solution or a suspension; combining the solution or the suspension with a carrier; removing the solvent; and subjecting the combination to grinding or milling. It would be apparent to one skilled in the art to select a suitable solvent or combination of solvents for dissolving or suspending the oily substance. Any volatile solvent is suitable. Suitable solvents include, but are not limited to, C₁₋₆ alcohols, polyethers, C₃₋₅ ketones, C₃₋₆ esters, C₄₋₅ ethers, or water. Preferably, the solvent is ethanol. More preferably, the solvent is an aqueous 95% ethanol solution. These solvents are also preferred when a poloxamer is used as the oily substance.

In another embodiment, the present invention encompasses a process for preparing a pharmaceutical excipient complex comprising melting an oily substance; combining the melted oily substance with a carrier; cooling the combination; and grinding or milling the combination. Suitable oily substances for use in this case include, but are not limited to, glyceryl behanate, bees wax, glyceril stearate, stearyl alcohol, and hydrogenated castor oil.

In another embodiment, the present invention encompasses a process for preparing a pharmaceutical excipient complex comprising combining a liquid oily substance with a carrier; and grinding or milling the combination. Suitable oily substances for use in this case include, but are not limited to, liquid triglycerides.

In a further embodiment, the present invention encompasses a stable pharmaceutical composition comprising an active pharmaceutical ingredient and a pharmaceutical excipient complex having at least one carrier intimately admixed with an oily substance. The API of the above stable composition has an assay of 95-105% of the labeled amount at the end of the storage period. As used herein, the term "storage period" means a shelf life at controlled conditions or four days at a temperature of 65°C and a relative humidity of 100 percent. The active pharmaceutical ingredient has low aqueous solubility. Active pharmaceutical ingredients with low solubility include, but are not limited to, albuterol, adapalene, doxazosin mesylate, mometasone furoate, ursodiol, amphotericin, enalapril maleate, felodipine, nefazodone hydrochloride, valrubicin, albendazole, conjugated estrogens, medroxyprogesterone acetate, nicardipine hydrochloride, zolpidem tartrate, amlodipine besylate, ethinyl estradiol, omeprazole, rubitecan, amlodipine besylate/benazepril hydrochloride, etodolac, paroxetine hydrochloride, paclitaxel, atovaquone, felodipine, podofilox, paricalcitol, betamethasone dipropionate, fentanyl, pramipexole dihydrochloride, Vitamin D₃, finasteride, quetiapine fumarate, alprostadil, candesartan, cilexetil, fluconazole, ritonavir, busulfan, carbamazepine, flumazenil, risperidone, carbemazepine, carbidopa/levodopa, ganciclovir, saquinavir, amprenavir, carboplatin, glyburide, sertraline hydrochloride, rofecoxib, carvedilol, halobetasol proprionate, sildenafil citrate, celecoxib, chlorthalidone, imiquimod, simvastatin, citalopram, ciprofloxacin, irinotecan hydrochloride, sparfloxacin, efavirenz, cisapride monohydrate, lansoprazole, tamsulosin hydrochloride, mofafinil, azithromycin, clarithromycin, letrozole, terbinafine hydrochloride, rosiglitazone maleate, diclofenac sodium, lomefloxacin hydrochloride, tirofiban hydrochloride, telmisartan, diazapam, loratadine, toremifene citrate, thalidomide, dinoprostone, mefloquine hydrochloride, trandolapril, docetaxel, mitoxantrone hydrochloride, tretinoin, etodolac, triamcinolone acetate, estradiol, ursodiol, nelfinavir mesylate, indinavir, beclomethasone dipropionate, oxaprozin, flutamide, famotidine, nifedipine, prednisone, cefuroxime, lorazepam, digoxin, lovastatin, griseofulvin, naproxen, ibuprofen, isotretinoin, tamoxifen citrate nimodipine, amiodarone, and alprazolam. Preferably, the active pharmaceutical ingredient is candesartan cilexetil.

In another embodiment, the present invention encompasses a process for preparing a stable pharmaceutical composition comprising combining at least one carrier with an oily substance to form a pharmaceutical excipient complex; grinding the pharmaceutical excipient complex; and admixing the ground pharmaceutical excipient complex with an active pharmaceutical ingredient to provide a stable pharmaceutical composition. In one embodiment, combination step combines the carrier and the oily substance using a wet granulation process, followed by drying of the granulate. Preferably, the active pharmaceutical ingredient has low aqueous solubility and is selected from the group consisting of albuterol, zolpidem tartrate, omeprazole, paclitaxel, quetiapine fumarate, alprostadil, candesartan, risperidone, carvedilol, celecoxib, ciprofloxacin, and alprazolam. More preferably, it is candesartan cilexetil.

Optionally, the process further comprises combining the pharmaceutical composition with other pharmaceutical excipients and the pharmaceutical excipients may be subjected to grinding.

In one example embodiment, the stable pharmaceutical composition of the present invention comprises candesartan cilexetil, starch, lactose monohydrate and a poloxamer, wherein at least one part of the starch and lactose monohydrate is coated with the poloxamer. Preferably, the poloxamer added is poloxamer 407.

Candesartan cilexetil can be prepared using any method known in the art. For example, according to US Publication No. 2005/0250827, candesartan cilexetil is prepared by providing cilexetil trityl candesartan; deprotecting cilexetil trityl candesartan in a mixture of water and methanol to obtain a residue of candesartan cilexetil; crystallizing the residue of candesartan cilexetil using methanol and toluene; and then recrystallizing the candesartan cilexetil in methanol to produce crystalline candesartan cilexetil.

The amount of candesartan cilexetil contained in a pharmaceutical composition for treating circulatory system diseases according to the present invention is not specifically restricted, however, the dose should be sufficient to treat, ameliorate, or reduce the symptoms associated with the circulatory system disease. The dosage of a pharmaceutical composition for treating circulatory system diseases according to the present invention will depend on the method of use, the age, sex, and condition of the patient. Preferably, about 4 mg, 8 mg, 16 mg or 32 mg of candesartan cilexetil may be contained in a dosage form.

The stable pharmaceutical composition of the present invention may be prepared by any conventional means such as, but not limited to, wet or dry granulation and direct compression. Preferably the preparation of the pharmaceutical composition undergoes at least one compression step. More preferably, it undergoes a direct compression process.

In a direct compression process, the process for preparing a pharmaceutical composition comprises combining a pharmaceutical excipient complex having at least one carrier and an oily substance with an active pharmaceutical ingredient, wherein the carrier is intimately admixed with the oily substance. Optionally, one or more other excipients are added to the pharmaceutical composition and the resulting combination is compressed into a solid pharmaceutical composition such as tablets, pills, granules, etc. Preferably, the solid pharmaceutical composition is compressed into a tablet.

In some cases, the carrier and/or the oily substance of the present invention may have a dual usage if present in large amounts. When this is the case, the carrier and the oily substance may also act as binders, fillers, stabilizers or solubilizing agents when incorporated into a pharmaceutical composition containing active pharmaceutical ingredients. However, for this dual activity to occur, the carrier and the oily substance must be present in the amount necessary to perform the function of a carrier or oily substance prior to acting as a binder, filler or stabilizers or solubilizing agents.

However, in some cases, this dual activity of the carrier and oily substance can cause difficulties. For example, in the case of candesartan cilexetil, the amount of the oily substance (e.g. poloxamer 407) needed to effectively stabilize the degradation of candesartan cilexetil may be in excess of requirement as regards its solubilizing activity. As can be seen in Example 3 of co-pending application SN 60/963,014, the use of 5.2 mg and 6.4 mg of poloxamer effectively stabilizes the API, and achieves a very high blood concentration and bioavailability of the drug. By using the pharmaceutical excipient complex of the present invention, the benefit of stabilization can be maintained, while at the same time reducing the solubilizing nature of the poloxamer, such that acceptable pharmacokinetics is achieved.

The pharmaceutical compositions of the present invention may further contain one or more additional excipients including, but not limited to, fillers, diluents, disintegrants, glidants, lubricants, further carriers, bulking agents, binders, wetting agents, and the like.

Suitable fillers and diluents include, but are not limited to, cellulose-derived materials like powdered cellulose, microcrystalline cellulose (e.g. Avicel®), microfine cellulose, methyl cellulose, ethyl cellulose, hydroxyethyl cellulose, hydroxypropyl cellulose, hydroxypropyl methylcellulose, carboxymethyl cellulose salts and other substituted and unsubstituted celluloses; starch; pregelatinized starch; lactose; talc; waxes; sugars; sugar alcohols like mannitol and sorbitol; acrylate polymers and copolymers; dextrates; dextrin; dextrose; maltodextrin; pectin; gelatin; inorganic diluents like calcium carbonate, dibasic calcium phosphate dihydrate, tribasic calcium phosphate, calcium sulfate, magnesium carbonate, magnesium oxide, sodium chloride and other diluents known to the pharmaceutical industry.

Solid pharmaceutical compositions of the present invention that are compacted into a dosage form, such as a tablet, may include an addition of a disintegrant to the composition. Suitable disintegrants include, but are not limited to, croscarmellose sodium (e.g. Ac Di Sol^{®}, Primellose^{®}), crospovidone (e.g. Kollidon^{®}, Polyplasdone^{®}), microcrystalline cellulose, polacrilin potassium, powdered cellulose, pregelatinized starch, sodium starch glycolate (e.g. Explotab^{®}, Primoljel^{®}) and starch.

Glidants can be added to improve the flowability of a solid composition before compaction and to improve the accuracy of dosing especially during compaction and capsule filling. Excipients that may function as glidants include, but are not limited to, colloidal silicon dioxide, magnesium trisilicate, powdered cellulose, and talc.

A lubricant may be added to the pharmaceutical compositions of the present invention to reduce adhesion and/or ease the release of the product from e.g. the die. Suitable lubricants include, but are not limited to, magnesium stearate, calcium stearate, glyceryl monostearate, glyceryl palmitostearate, hydrogenated castor oil, hydrogenated vegetable oil, mineral oil, polyethylene glycol, sodium lauryl sulfate, sodium stearyl fumarate, stearic acid, talc and zinc stearate.

Additional carriers include, but are not limited to, lactose, white sugar, sodium chloride, glucose, urea, starch, calcium carbonate, kaolin, crystalline cellulose, and silicic acid.

Binders include, but are not limited to, water, ethanol, propanol, simple syrup, glucose solutions, starch solutions, gelatin solutions, carboxymethyl cellulose, shelac, methyl cellulose, potassium phosphate, and polyvinylpyrrolidone. Suitable binders include, but are not limited to, acacia gum, pregelatinized starch, sodium alginate, glucose and other binders used in wet or dry granulation and in direct compression tableting processes.

Other excipients that may be incorporated into the formulation include, but are not limited to, preservatives, surfactants, antioxidants, or any other excipient commonly used in the pharmaceutical industry.

The pharmaceutical composition of the invention may take any form but it is preferably a solid composition. More preferably, the pharmaceutical composition of the invention is a compressed solid composition. Suitable solid dosage forms include, but are not limited to, tablets, capsules, powders, and sachets.

Preferably, the dosage form is a tablet. For example, the pharmaceutical composition of the present invention may be a compressed granulate in the form of a tablet or a tablet formed by direct compression of the tablet components. A tablet formed by direct compression is the most preferred.

Many tablets today are coated after being pressed. A coating may be applied to hide the taste of the tablet's components, to make the tablet smoother and easier to swallow, and to make it more resistant to the environment, extending its shelf life or altering its solubility. Tablets may be coated with a variety of commonly known coating materials, for example, tablets may be coated with sugar, gelatin film, or enteric coating. There are also double layered tablets and multi-layered tablets.

A coated tablet may have a coating on the surface of the tablet or may be a tablet comprising a powder or granules with an enteric-coating. The enteric coated powder forms may have coatings including, but not limited to, phthalic acid cellulose acetate, hydroxypropylmethyl-cellulose phthalate, polyvinyl alcohol phthalate, carboxymethylethylcellulose, a copolymer of styrene and maleic acid, a copolymer of methacrylic acid and methyl methacrylate, and the like, and if desired, they may be employed with suitable plasticizers and/or extending agents.

When the pharmaceutical composition is in the dosage form of a capsule, the capsule may contain the pharmaceutical composition of the invention in a form of uncompressed or compressed granulates or powder mixes, etc. The capsules may be covered with either a hard shell or a soft shell. The shells may be made from, but not limited to gelatin and optionally contain a plasticizer such as glycerin and sorbitol, and an opacifying agent or colorant.

For the purpose of shaping the pharmaceutical composition in the form of suppositories, any commonly known excipient used in the art may be used. For example, excipients include, but are not limited to, polyethylene glycols, coconut butter, higher alcohols, esters of higher alcohols, gelatin, and semi-synthesized glycerides.

Methods of administration of a pharmaceutical composition for treating circulatory system diseases of the present invention are not specifically restricted, and can be administered in various preparations depending on the age, sex, and symptoms of the patient. Suitable routes for administrating a pharmaceutical composition may include, but not limited to, oral, buccal, and rectal administration. Although the most suitable administration in any given case will depend on the nature and severity of the condition being treated, the most preferred route of administration of the present invention is oral. The dosages may be conveniently presented in unit dosage form and prepared by any of the methods commonly known in the pharmaceutical arts.

Having described the invention with reference to certain preferred embodiments, other embodiments will become apparent to one skilled in the art from consideration of the specification. The invention is further defined by reference to the following examples describing in detail the preparation of the pharmaceutical excipient complex and the pharmaceutical composition. It will be apparent to those skilled in the art that many modifications, both to materials and methods, may be practiced without departing from the scope of the invention.

### EXAMPLES

### Example 1

A pharmaceutical excipient complex according to the present invention was prepared as follows.

Starch (starch 1500) (50.0 mg, 53% w/w) and lactose monohydrate (200 mesh) (38.0 mg, 40.2% w/w) were mixed in a high shear mixer. Poloxamer 407 (Lutrol^{®} F127) (6.4 mg, 6.8% w/w) was mixed in 95% ethanol solution until a solution was obtained. The resulting solution was added into the high shear mixer and mixed. The resulting wet granulate was then dried in a fluid bed dryer and then milled with a conical mill (Quadro).

### Example 2

A pharmaceutical composition according to the present invention was prepared.

The pharmaceutical excipient complex prepared in Example 1 was mixed with candesartan cilexetil. The resulting mixture was then compressed to form a tablet.

### Example 3

Another pharmaceutical excipient complex according to the present invention was prepared as follows.

Starch (starch STA-RX 1500) (6,300 g, 33.3% w/w) and lactose monohydrate (Pharmatose DCL 14) (11,844 g, 62.7% w/w) were mixed in a high shear mixer. Poloxamer 407 (Lutrol^{®} F127) (756 g, 4% w/w) was mixed in a 95% USP ethanol solution (3,500 g) to provide the granulate solution, and added to the starch and lactose monohydrate mixture. The resulting combination was mixed, and then dried in a fluid bed dryer and then milled with a conical mill (Quadro).

### Example 4

A pharmaceutical composition according to the present invention was prepared as follows.

Lactose monohydrate (38,088 g, 58.8% w/w), candesartan cilexetil (5,760 g, 8.9% w/w), Providone (PVP K-30) (2,880 g, 4.4% w/w) and carboxymethylcellulose calcium EP (810 g, 1.3% w/w) were combined in a dry blender. A mixture of carboxymethylcellulose calcium EP (360 g, 0.5% w/w) and Color Ferric Oxide Red E172 (54 g, 0.08% w/w) was added into the dry blender, as well as the pharmaceutical excipient complex (16,200 g, 25% w/w) prepared in Example 3. The above ingredients were mixed together for about 10 minutes, and then Magnesium Stearate Ph. Eur. (648 g, 1% w/w) was added into the dry blender, and the mixing continued for about 5 minutes.

### Example 5

A pharmaceutical composition according to the present invention was prepared as follows.

Starch (starch 1500) (50.0 mg) and lactose monohydrate (200 mesh) (38.0 mg) were mixed in a high shear mixer. Poloxamer 407 (Lutrol^{®} F127) (3.2 mg) was mixed in 95% ethanol solution until a solution was obtained. The resulting solution was added into the high shear mixer and mixed. The resulting wet granulate was then dried in a fluid bed dryer and then milled with a conical mill (Quadro). Candesartan cilexetil (32 mg), Spray dried Lactose (171 mg), Providone (PVP K-30) (16 mg) were mixed in with the resulting granulate. The above ingredients were mixed together and then Magnesium Stearate Ph. Eur. (3.1 mg) was added and mixing continued. The resulting mixture was then compressed to form a tablet.

### Results

The stability of the tablet produced according to Example 5 was studied. At the initial time, the impurity desethyl-CNS was present in 0.07 % by the total weight of the pharmaceutical composition. After storage for four days at a temperature of 70°C and a relative humidity of 100 percent, the amount had increased to 0.6% by the total weight of the pharmaceutical composition. At the initial time, impurities other than desethyl-CNS and 2-N-ethyl CNS were not detected; however, after the storage period impurities other than desethyl-CNS and 2-N-ethyl CNS were found in 0.1 % by weight of the total weight of the pharmaceutical composition. Based on this data, it was concluded that candesartan cilexetil did not degrade significantly when tabletted with the excipient complex of the invention

## Claims

1. A pharmaceutical excipient complex comprising at least one carrier intimately admixed with an oily substance.

2. The pharmaceutical excipient complex of claim 1, wherein the carrier is selected from the group consisting of lactose, white sugar, glucose, starch, calcium carbonate, kaolin, crystalline cellulose, maltose, mannitol, sorbitol, hydroxypropyl cellulose, maltodextrin, dibasic calcium phosphate and silicon dioxide.

3. The pharmaceutical excipient complex of claim 2, wherein the carrier is starch, lactose monohydrate, mannitol, sorbitol, or a mixture thereof.

4. The pharmaceutical excipient complex of any one or more of Claims 1 to 3, wherein the oily substance is selected from the group consisting of waxes, polymers, block co-polymers, stabilizers, surfactants, fats, fatty acids, and mixtures thereof.

5. The pharmaceutical excipient complex of any one or more of Claims 1 to 4, wherein the oily substance is selected from the group consisting of poloxamer, glyceryl behenate, bees wax, glyceryl stearate, stearyl alcohol, hydrogenated castor oil liquid triglyceride, and mixture thereof.

6. The pharmaceutical excipient complex of claim 5, wherein the oily substance is a poloxamer or polyoxyethylene-polyoxypropylene copolymer having an average molecular weight of about 9800 to about 14000.

7. The pharmaceutical excipient complex according to any one or more of claims 1-6, wherein the weight ratio between the oily substance and the carrier is in the range of about 1:100 to about 1:1.

8. A pharmaceutical composition comprising an active pharmaceutical ingredient of low aqueous solubility and a pharmaceutical excipient complex according to any one or more of Claims 1 to 7.

9. The pharmaceutical composition of Claim 8, wherein the active pharmaceutical ingredient is selected from the group consisting of albuterol, zolpidem tartrate, omeprazole, paclitaxel, quetiapine fumarate, alprostadil, candesartan, risperidone, carvedilol, celecoxib, ciprofloxacin, and alprazolam.

10. The pharmaceutical composition of Claim 9, wherein the active pharmaceutical ingredient is candesartan cilexetil.

11. A stable pharmaceutical composition comprising an active pharmaceutical ingredient and a pharmaceutical excipient complex according to any one or more of Claims 1 to 7.

12. A stable pharmaceutical composition comprising candesartan cilexetil and a pharmaceutical excipient complex according to any one or more of Claims 1 to 7.

13. A process for preparing a pharmaceutical excipient complex comprising dissolving or suspending the oily substance in a solvent to form a solution or a suspension; combining the solution or the suspension with a carrier; removing the solvent; and subjecting the combination to grinding or milling.

14. The process of claim 13, wherein the oily substance is selected from the group consisting of waxes, polymers, block co-polymers, stabilizers, surfactants, fats, fatty acids, and mixtures thereof.

15. The process according to any one or more of Claims 13 to 14, wherein the solvent is selected from the group consisting of C₁₋₆ alcohols, polyethers, C₃₋₅ ketones, C₃₋₆ esters, C₄₋₅ ethers, and water.

16. The process of claim 15, wherein the solvent is ethanol.

17. A process for preparing a pharmaceutical excipient complex comprising melting an oily substance; combining the melted oily substance with a carrier; cooling the combination; and grinding or milling the combination.

18. The process of claim 17, wherein the oily substance is glyceryl behanate, bees wax, glyceryl stearate, stearyl alcohol, or hydrogenated castor oil.

19. A process for preparing a pharmaceutical excipient complex comprising combining a liquid oily substance with a carrier; and grinding or milling the combination.

20. The process of claim 19, wherein the oily substance is liquid triglyceride.

21. The process of any one or more of Claims 13 to 20, wherein the carrier is selected from the group consisting of lactose, white sugar, glucose, starch, calcium carbonate, kaolin, crystalline cellulose, maltose, mannitol, sorbitol, hydroxypropyl cellulose, maltodextrin, dibasic calcium phosphate and silicon dioxide.

22. The process of any one or more of Claims 13 to 21 wherein the pharmaceutical excipient complex is as defined in any one or more of Claims 1 to 7.

23. A process for preparing a pharmaceutical composition comprising a pharmaceutical excipient complex comprising combining at least one carrier with an oily substance; subjecting the combination to grinding or milling; and admixing the combination with an active pharmaceutical ingredient.

24. A process according to Claim 23 wherein the pharmaceutical excipient complex is as defined in any one or more of Claims 1 to 7.

25. The process of any one or more of Claims 23 to 24, wherein the carrier and the oily substance are combined by wet granulation.

26. The process of any one or more of Claims 23 to 25, wherein the active pharmaceutical ingredient is selected from the group consisting of albuterol, zolpidem tartrate, omeprazole, paclitaxel, quetiapine fumarate, alprostadil, candesartan, risperidone, carvedilol, celecoxib, ciprofloxacin, and alprazolam.

27. The process according to any one or more of Claims 23 to 26, wherein the carrier is selected from the group consisting of lactose, white sugar, glucose, starch, calcium carbonate, kaolin, crystalline cellulose, maltose, mannitol, sorbitol, hydroxypropyl cellulose, maltodextrin, dibasic calcium phosphate and silicon dioxide.

28. The process according to any one or more of Claims 23 to 27, wherein the oily substance is selected from the group consisting of waxes, polymers, block co-polymers, stabilizers, surfactants, fats, fatty acids, and mixtures thereof.

29. The process according to any one or more of Claims 23 to 28, wherein the process further comprises compressing the pharmaceutical composition into a solid dosage form.

30. The process of claim 29, wherein the compressing step is a direct compression process.

31. Use of an active pharmaceutical ingredient and a pharmaceutical excipient complex, as defined in any one or more of Claims 1 to 7 in the manufacture of a medicament for treating and/or preventing a patient suffering from a disease.

32. Use according to Claim 31, wherein the active pharmaceutical ingredient is selected from the group consisting of albuterol, zolpidem tartrate, omeprazole, paclitaxel, quetiapine fumarate, alprostadil, candesartan, risperidone, carvedilol, celecoxib, ciprofloxacin, and alprazolam.

33. Use according to Claim 32 wherein the active pharmaceutical ingredient is candesartan cilexetil and the disease is a circulatory system disease, preferably hypertension.

34. A pharmaceutical composition according to any one or more of Claims 8 to 12 for use as a medicament for treating and/or preventing a patient suffering from a disease.

35. A pharmaceutical composition according to Claim 34 wherein the active pharmaceutical ingredient is selected from the group consisting of albuterol, zolpidem tartrate, omeprazole, paclitaxel, quetiapine fumarate, alprostadil, candesartan, risperidone, carvedilol, celecoxib, ciprofloxacin, and alprazolam.

36. A pharmaceutical composition according to Claim 35 wherein the active pharmaceutical ingredient is candesartan cilexetil and the disease is a circulatory system disease, preferably hypertension.
